(19) Europäisches Patentamt
European Patent Office
Office européen des brevets

(11) Veröffentlichungsnummer: **0 332 981 B1**

(12) **EUROPÄISCHE PATENTSCHRIFT**

(45) Veröffentlichungstag der Patentschrift: **15.06.94**

(51) Int. Cl.$^5$: **C07C 69/738**, C07C 67/313

(21) Anmeldenummer: **89103946.3**

(22) Anmeldetag: **07.03.89**

# (54) Verfahren zur Herstellung von alpha-Ketocarbonsäureestern.

(30) Priorität: **15.03.88 DE 3808512**

(43) Veröffentlichungstag der Anmeldung:
**20.09.89 Patentblatt 89/38**

(45) Bekanntmachung des Hinweises auf die Patenterteilung:
**15.06.94 Patentblatt 94/24**

(84) Benannte Vertragsstaaten:
**BE CH DE FR GB IT LI NL**

(56) Entgegenhaltungen:
**EP-A- 0 100 117**
**EP-A- 0 262 532**
**EP-A- 0 317 911**
**DE-A- 2 533 335**

(73) Patentinhaber: **BASF Aktiengesellschaft**
**Carl-Bosch-Strasse 38**
**D-67063 Ludwigshafen(DE)**

(72) Erfinder: **Goetz, Norbert, Dr.**
**Schoefferstrasse 25**
**D-6520 Worms 1(DE)**
Erfinder: **Hoelderich, Wolfgang, Dr.**
**Mannheimer Strasse 18 c**
**D-6710 Frankenthal(DE)**
Erfinder: **Hupfer, Leopold, Dr.**
**Waltershoehe 3**
**D-6701 Friedelsheim(DE)**

EP 0 332 981 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft ein Verfahren zur Herstellung von α-Ketocarbonsäureestern aus Glycidsäureestern in Gegenwart von Zeolithen und/oder Phosphaten und/oder Phosphorsäure bzw. Borsäure auf Trägermaterial und/oder sauren Metalloxiden und Verwendung dieser α-Ketocarbonsäureester zur Herstellung von Phenylessigsäureestern an den gleichen Katalysatoren.

Die als Vorprodukte benutzten Glycidester können in einfacher Weise aus Carbonylverbindungen, z.B. Aldehyden und Ketonen und α-Chlorcarbonsäureestern hergestellt werden. Man hat damit eine sehr breit anwendbare Methode, mit der man fast jedes gewünschte Substitutionsmuster einstellen kann.

Die Synthese der α-Ketocarbonsäuren bzw. deren Estern erfolgte bisher über aufwendige Wege, wie beispielsweise über Grignard-Reaktionen mit Oxalsäureestern.

Das Standardverfahren zur Herstellung von Phenylessigestern setzt sich zusammen aus der Umsetzung von Benzylhalogeniden mit Kaliumcyanid zu Benzylcyaniden und deren hydrolytische Spaltung zu Phenylessigestern - ein mehrstufiges Verfahren, bei dem mit hochgiftigem Kaliumcyanid gearbeitet werden muß.

Was die weitere Verwendung sowohl der α-Ketocarbonsäureester als auch der Phenylessigsäureester anbetrifft, so gibt es eine ganze Reihe von Verwendungen: α-Ketocarbonsäuren bzw. deren Derivate können zur Herstellung von herbiziden Triazinonen (EP 58.885, DE-OS 31 06 707) oder zur Gewinnung von L-Aminosäuren (DE-OS 36 14 586) eingesetzt werden.

Aus der DE-A-25 33 335 ist ein Verfahren zur Herstellung von Brenztraubensäurederivaten durch Erhitzen von Glycidylestern in Gegenwart eines elektrophilen Reagenzes bekannt. Als elektrophile Reagenzien werden nur Homogenkatalysatoren beschrieben (s. Seite 5, 2. Absatz).

Es ist bekannt, daß man Epoxide in Gegenwart von Zeolithen zu Carbonylverbindungen umlagern kann.

In EP 100 117 wird die Reaktion von Styroloxid und von am aromatischen Kern alkyl- bzw. alkoxylsubstituierten Styroloxiden an einem Titanhaltigen Zeolithen zu Phenylacetaldehyden in der flüssigen Phase bei 30 bis 100 °C beschrieben. Der hierzu eingesetzte Katalysator muß aufwendig aus teuren hochreinen Ausgangsstoffen wie Tetraalkylorthosilikat, Tetraalkylorthotitanat und Tetrapropylammoniumhydroxid hergestellt werden. Hohe Umsätze werden nur bei Reaktion in Lösungsmitteln wie Methanol oder Aceton bei 30 bis 100 °C in der Flüssigphase und bei Verweilzeiten von 1 bis 1,5 h erzielt. Dies verursacht erhöhte Destillations- und Betriebskosten. Weiterhin ist die Reaktion an titanhaltigen Zeolithen nur bei Styroloxid und alkylierten bzw. alkoxylierten Styroloxiden möglich.

Es sind auch andere Arbeiten zur Umlagerung von Epoxiden zu Carbonylverbindungen bekannt. Cyclodecanon beispielsweise wird an Pd- oder Rd-dotierten $Al_2O_3$ aus Epoxycyclododecan erhalten (Neftekhimiya 16 (1976), 250-254). In dieser Arbeit wird ausdrücklich darauf hingewiesen, daß Zeolithe für diese Reaktion ungeeignet sind. Auch der Einsatz von A-Zeolithen für die Umlagerung von Butylenoxid zu Butyraldehyd (55 bis 72 %) ist beschrieben (Hokkaido Daigaku Kogarubu Hokoku 67 (1973), 171-178). Die Selektivität läßt zu wünschen übrig. Auch läßt sich der A-Zeolith-Katalysator nach seiner Desaktivierung durch Koks schwer regenerieren, da bei den hierfür notwendigen Temperaturen von etwa 500 °C die Kristallstruktur dieses Zeolithen zerstört wird. Weiterhin ist es für die Umsetzung von Propylenoxid zu Aceton oder Propionaldehyd an alkalidotierten X-Zeolithen notwendig, in Abwesenheit stark acider Zentren zu arbeiten (Waseda Daigaku Rikogaku Kenkyusho Hokoku 67 (1974), 26-29).

Es bestand die Aufgabe, α-Ketocarbonsäureester und Phenylessigsäureester auf einfachem Wege aus wohlfeilen Glycidsäureestern herzustellen, wobei möglichst hoher Umsatz, hohe Selektivität und lange Standzeit der Katalysatoren angestrebt wird.

Es wurde nun gefunden, daß man bei der Herstellung von α-Ketocarbonsäureestern der Formel (I)

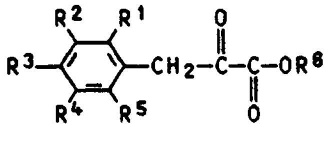

(I),

in der $R^1$ bis $R^5$ untereinander gleich oder verschieden sind und Wasserstoff oder geradkettige bzw. verzweigte Alkyl-, Alkenyl-, Hydroxyl-, Alkoxyl- und/ oder Halogenreste bedeuten und $R^6$ für einen Niederalkylrest steht, die Nachteile der bisherigen Synthesewege und bisher verwendeten Katalysatoren vermeidet, wenn man Glycidsäureester der Formel II

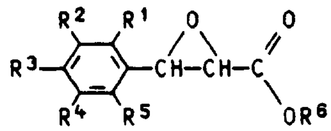

(II),

in der $R^1$ bis $R^6$ obige Bedeutung haben, in Gegenwart von Zeolithen und/oder Phosphaten und/oder Phosphorsäure bzw. Borsäure auf Trägermaterial und/oder sauren Metalloxiden als Katalysatoren umsetzt.

Die $\alpha$-Ketocarbonsäureester der Formel (I) können zur Herstellung von Phenylessigsäureester der Formel (III)

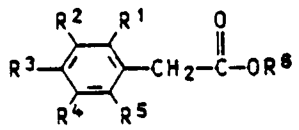

(III),

durch Decarbonylierung bei Temperaturen über 300 °C in Gegenwart der oben genannten Katalysatoren verwendet worden.

Es ist auch möglich, Glycidsäureester der Formel (II) in einer Stufe direkt in die Phenylessigsäurester der Formel (III) überzuführen, ohne die $\alpha$-Ketocarbonsäureester der Formel (I) zu isolieren, wenn die Reaktionstemperatur gleich oder großer 350 °C beträgt.

Als Reste $R^1$, $R^2$, $R^3$, $R^4$ und $R^5$ der Formeln (I), die untereinander gleich oder verschieden sein können, sind Wasserstoff oder geradkettige bzw. verzweigte Alkylreste mit 1 bis 8 Kohlenstoffatomen, insbesondere 1 bis 4 Kohlenstoffatomen wie Methyl-, Ethyl-, n/i-Propyl-, n-/i-/t-Butyl-, n-Hexyl oder geradkettige bzw. verzweigte Alkenylreste mit 1 bis 8 Kohlenstoffatomen, insbesondere 1 bis 4 Kohlenstoffatomen wie Ethenyl-, Propenyl-, Butenyl- oder Hydroxylreste oder geradkettige bzw. verzweigte Alkoxyreste mit 1 bis 8 Kohlenstoffatomen, insbesondere 1 bis 4 Kohlenstoffatomen wie Methoxy-, Ethoxy-, Propoxy-, Butoxy- oder Cycloalkylreste wie Cyclopentyl- und Cyclohexyl-, Cyclohexenyl- oder Halogenreste wie F, Cl-, Trifluormethyl, Trichlormethyl, Trifluormethoxy, Monofluormethyl, Fluorethyl, Fluorpropyl geeignet.

Unter $R^6$ versteht man unverzweigte Niederalkylreste insbesondere mit 1 bis 4 Kohlenstoffatomen wie Methyl-, Ethyl-, n-Propyl- und n-Butylreste.

Als Katalysatoren für das erfindungsgemäße Verfahren werden acide zeolithische Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen. die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Für das erfindungsgemäße Verfahren werden als Katalysatoren insbesondere Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe verwendet. In diese Gruppe von Zeolithen gehören auch die sogenannten "ultrastabilen" Zeolithe des Faujasittyps, d.h, dealuminierte Zeolithe. Verfahren zur Herstellung solcher Zeolithe sind beschrieben in "Catalysis by Zeolites", Band 5 aus "Studies in Surface Science and Catalysis" ed. 8. Imelik et al. Elsevier Scientific Publishing Comp., 1980, S. 203 und "Crystal Structures of Ultra-stable Faujasites" Advances in Chemistry Series Nr. 101, American Chemical Society Washington, DC, S. 226ff (1971) und in US-PS 4 512 961.

Vorteilhaft verwendet man Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzungen aufweisen. Es handelt sich hierbei um Alumino-, Boro-. Eisen-, Beryllium-, Gallium-, Chrom-, Arsen-, Antimon- und Wismutsilikatzeolith oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Ausgangsmengen ein $SiO_2/Al_2O_3$-Verhältnis von 10 bis 40.000. Auch kann man derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter autogenem Druck synthetisiert, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch isotaktische Zeolithe nach EP 34 727 und EP 46 504 sind geeignet, Solche Borosilikatzeolithe können auch hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhalt man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Zu den verwendbaren siliciumreichen Zeolithen ($SiO_2/Al_2O_3 \geqq 10$) gehören auch die sog. ZSM-Typen, Ferrierit, NU-1 und Silicalit® (ein Molekularsieb, ein sog. Silica Polymorph).

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhaltnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino- bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose. Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor. sondern z.B. in der Na-Form, dann kann diese durch Ionenaustausch. z.B. mit Ammoniumionen und anschließende Calcinierung oder durch Behandlung mit Säuren vollkommen oder partiell in die gewünschte H-Form überführt werden.

Wenn bei der Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht z.B. darin, daß man die unverformten oder verformten Zeolithe mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4, bis 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn. Seltene Erdmetalle wie La, Ce, Pr, Nd, Fr, Yb und U eingesetzt.

Zweckmäßigerweise führt man die Dotierung so durch, daß man die verformten Zeolithe in einem Steigrohr vorlegt und bei 20 bis 100 °C eine wäßrige oder ammoniakalische Lösung eines Halogenids oder eines Nitrats der beschriebenen Metalle darüberleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen besteht darin, daß man das zeolithische Material mit einem Halogenid, einem Nitrat oder einem Oxid der beschriebenen Metalle in wäßriger, alkoholischer oder ammoniakalischer Lösung imprägniert. Sowohl an einen Ionenaustausch als auch an eine Imprägnierung schließt sich

zumindest eine Trocknung, wahlweise eine abermalige Calcinierung an.

Eine mögliche Ausführungsform besteht darin, daß man $Cu(NO_3)_2x3H_2O$ oder $Ni(NO_3)_2x6H_2O$ oder $Ce(NO_3)_3x6H_2O$ oder $La(NO_3)_2x6H_2O$ oder $Cs_2CO_3$ in Wasser löst. Mit dieser Lösung wird der verformte oder unverformte Zeolith eine gewisse Zeit, etwa 30 Minuten, getränkt. Die überstehende Lösung wird am Rotationsverdampfer von Wasser befreit. Danach wird der getränkte Zeolith bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, eine wäßrige $Ni(CO_3)_2$-Lösung oder ammoniakalische $Pd(NO_3)_2$-Lösung herzustellen und darin die reinen pulverförmigen Zeolithe bei 40 bis 100 °C unter Rühren etwa 24 h aufzuschlämmen. Nach Abfiltrieren. Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch der in der H-Form oder Ammonium-Form oder Alkali-Form vorliegenden Zeolithe kann so vorgenommen werden, daß man die Zeolithe in Strängen oder Pellets in einer Kolonne vorlegt und darüber eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert. Bei manchen metalldotierten Zeolithen wie den Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Sauren wie Salzsäure, Flußsäure und Phosphorsäure und/oder Wasserdampf unterwirft. Dabei geht man vorteilhaft so vor, daß man Zeolithe in Pulverform mit 1 n Phosphorsäure 1 Stunde bei 80 °C behandelt. Nach der Behandlung wird mit Wasser gewaschen, bei 110 °C/16 h getrocknet und bei 500 °C/20 h calciniert.

Nach einer anderen Arbeitsweise behandelt man Zeolithe vor oder nach ihrer Verformung mit Bindemitteln, z.B. 1 bis 3 Stunden bei Temperaturen von 60 bis 80 °C mit einer 3 bis 25 gew.%igen, insbesondere 12 bis 20 gew.%igen wäßrigen Salzsäure. Anschließend wurde der so behandelte Zeolith mit Wasser gewaschen, getrocknet und bei 400 bis 500 °C calciniert.

Eine besondere Ausführungsform für die Säurebehandlung besteht darin, daß man das zeolithische Material vor seiner Verformung bei erhöhter Temperatur mit 0,001 n bis 2 n, vorzugsweise 0,05 n bis 0,5 n Flußsäure durch Erhitzen unter Rückfluß über einen Zeitraum von im allgemeinen 0,5 bis 5, vorzugsweise 1 bis 3 Stunden, behandelt. Nach Isolierung durch Abfiltrieren und Auswaschen des zeolithischen Materials wird dieses zweckmäßig bei Temperaturen von 100 bis 160 °C getrocknet und bei Temperaturen von 450 bis 600 °C calciniert. Gemäß einer anderen bevorzugten Ausführungsform für die Säurebehandlung wird das zeolithische Material nach einer Verformung mit Bindemittel bei erhöhter Temperatur, zweckmäßig bei Temperaturen von 50 bis 90 °C, vorzugsweise 60 bis 80 °C, über einen Zeitraum von 0.5 bis 5 h mit 12 bis 20 gew. %iger Salzsäure, behandelt. Anschließend wird das zeolithische Material ausgewaschen und zweckmäßig bei Temperaturen von 100 bis 160 °C getrocknet und bei Temperaturen von 450 bis 600 °C calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert.

Weitere Katalysatoren für das Verfahren sind Phosphate, insbesondere Aluminiumphosphate, Siliciumaluminiumphosphate, Siliciumeisenaluminiumphosphate, Cerphosphate, Zirkonphosphate, Borphosphate, Eisenphosphate oder deren Gemische.

Als Aluminiumphosphat-Katalysatoren werden für das Verfahren insbesondere unter hydrothermalen Bedingungen synthetisierte Aluminiumphosphate, die Zeolithstruktur haben, eingesetzt.

Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP 132 708, US 4 310 440 und US 4 473 663 beschrieben.

Beispielsweise $AlPO_4$-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und danach bei etwa 150 °C 20 bis 60 h unter autogenem Druck in einem Autoklaven umsetzt. Das abfiltrierte $AlPO_4$ wird bei 100 bis 160 °C getrocknet und bei 450 bis 550 °C calciniert.

$AlPO_4$-9 (APO-9) wird aus Orthophosphorsäure und Pseudoboehmit in wäßriger DABCO-Lösung (1,4-Diazabicyclo-(2,2,2)octan) bei etwa 200 °C unter autogenem Druck während 200 bis 400 h synthetisiert.

Die Synthese des $AlPO_4$-21 (APO-21) erfolgt aus Orthophosphorsäure und Pseudoboehmit in wäßriger Pyrrolidon-Lösung bei 150 bis 200 °C unter autogenem Druck während 50 bis 200 h.

Die für das erfindungsgemäße Verfahren eingesetzten Siliciumaluminiumphosphate sind z.B. SAPO-5, SAPO-11, SAPO-31 und SAPO-34. Die Synthese dieser Verbindung ist in EP 103 117, US 4 440 871 beschrieben. SAPO's werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200 °C während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C.

Als Phosphatkatalysatoren kann man bei dem Verfahren auch gefällte Aluminiumphosphate einsetzen. Ein derartiges Aluminiumphosphat wird beispielsweise hergestellt, indem 92 g Diammoniumhydrogenphosphat in 700 ml Wasser gelöst werden. Zu dieser Lösung wird 260 g $Al(NO_3)_3xH_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60 °C/16 h getrocknet.

Geeignete Borphosphate für das erfindungsgemäße Verfahren kann man beispielsweise durch Mischen und Kneten von konzentriertor Borsäure und Phosphorsäure und durch anschließende Trocknung und Calcination in Inertgas-, Luft- oder Dampfatmosphäre bei 250 bis 650 °C vorzugsweise 300 bis 500 °C herstellen.

Auf diese Phosphate können auch Imprägnierungen (Tränken und Aufsprühen) oder in manchen Fällen auch durch Ionenaustausch Modifizierungskomponenten, wie oben für die Zeolithe beschrieben, aufgebracht werden. Wie bei den Zeolithkatalysatoren kann auch eine Modifizierung mit Säuren erfolgen.

Geeignete saure Katalysatoren sind z.B. die sauer wirkenden Oxide von Elementen der III. und IV. Hauptgruppe sowie der IV. bis VI. Nebengruppe des periodischen Systems, insbesondere Oxide wie Siliciumdioxid in Form von Kieselgel, Kieselgur, Quarz, weiterhin Titandioxid, Zirkondioxid, Phosphoroxide, Vanadiumoxide, Nioboxide, Boroxide, Chromoxide, Molybdänoxide, Wolframoxide oder Bims oder Gemische dieser Oxide. Diese Oxide können auch durch Aufbringung von Modifizierungskomponenten, wie oben für die Zeolithkatalysatoren beschrieben, dotiert werden. Auch durch eine Behandlung mit Säuren ist, wie bei den Zeolithkatalysatoren eine Möglichkeit der Modifizierung gegeben.

Auch mit Phosphorsäure oder Borsäure getränkte Katalysatoren können eingesetzt werden. Phosphorsäure oder Borsäure wird z.B. auf $SiO_2$-, $Al_2O_3$- oder Bimsträger, z.B. durch Auftränken oder Versprühen aufgebracht. Ein phosphorsäurehaltiger Katalysator kann beispielsweise durch Auftränken von $H_3PO_4$- oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden; danach schließt sich eine Trocknung und meist eine Calcination an. Phosphorsäure kann auch in einer Imprägniermühle auf das Trägermaterial aufgesprüht werden.

Die hier beschriebenen Katalysatoren können wahlweise als 2- bis 4 mm-Stränge oder als Tabletten mit 3 bis 5 mm Durchmesser oder als Splitt mit Teilchengrößen von 0,1 bis 0,5 mm oder als Wirbelkontakt eingesetzt werden.

Die erfindungsgemäße Umwandlung wird bevorzugt in der Gasphase bei 100 bis 500 °C, vorzugsweise bei 200 bis 350 °C, insbesondere 250 bis 300 °C, und einer Belastung WHSV = 0,1 bis 20 $h^{-1}$, bevorzugt 0,5 bis 5 $h^{-1}$ (g Ausgangsstoff/g Katalysator und Stunde) ausgeführt. Die Reaktion kann man in einem Festbett oder auch im Wirbelbett ausführen.

Es ist auch möglich, die Reaktion in der Flüssigphase (Suspension-, Riesel- oder Sumpffahrweise) bei Temperaturen zwischen 50 und 200 °C durchzuführen.

Das Verfahren kann bei Normaldruck, bei vermindertem oder erhöhtem Druck diskontinuierlich, vorzugsweise kontinuierlich, durchgeführt werden.

Schwerflüchtige oder feste Ausgangsstoffe werden in gelöster Form z.B. in THF-, Toluol- oder Petrolether-Lösung eingesetzt. Allgemein ist eine Verdünnung der Ausgangsstoffe mit Lösungsmitteln oder mit Inertgasen wie $N_2$, Ar, $H_2O$-Dampf möglich.

Nach dar Umsetzung werden die entstandenen Produkte durch übliche Verfahren, z.B. durch Destillation aus dem Reaktionsgemisch isoliert; nichtumgesetztes Ausgangsgemisch wird gegebenenfalls, zurückgeführt.

Beispiele 1 bis 15

Die Reaktionen in der Gasphase werden unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch.

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit einem Verformungshilfsmittel 2 mm-Stränge hergestellt, die bei 110 °C/16 h getrocknet und bei 500 °C/24 h calciniert werden.

Katalysator B

Ein Aluminosilikatzeolith vom Pentasil-Typ wird unter hydrothermalen Bedingungungen bei autogenem Druck und 150 °C aus 65 g hochdispersem $SiO_2$, 20,3 g $Al_2(SO_4)_3x18\ H_2O$ in 1 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Aluminosilikatzeolith enthält 91,6 Gew.% $SiO_2$ und 4,6 Gew.% $Al_2O_3$. Der Katalysator wird mit einem Verformungshilfsmittel zu 2 mm-Strängen verformt, bei 110 °C/16 h getrocknet und bei 500 °C/24 h calciniert.

Katalysator C

Katalysator C wird erhalten, indem man die Stränge des Katalysators A mit einer wäßrigen $Cs_2CO_3$-Lösung imprägniert, danach bei 130 °C/2 h trocknet und bei 540 °C/2 h calciniert. Der Cs-Gehalt beträgt 0.6 Gew.%.

Katalysator D

Katalysator D wird wie Katalysator C hergestellt, wobei jedoch $Cs_2CO_3$ durch $Ce(NO_3)_2$ ersetzt wird. Der Ce-Gehalt beträgt 1,8 Gew.%.

Katalysator E

Die Synthese von $AlPO_4$-5 (APO-5) erfolgt durch Zusammenrühren von 200 g 95 %ige Phosphorsäure gelöst in 325 g $H_2O$, 136 g Boehmit und 678 g Tetrapropylammoniumhydroxid (30 %ig) und anschließende Reaktion bei 150 °C unter autogenem Druck während 43 Stunden. Das bei 120 °C getrocknete und bei 500 °C/16 h calcinierte Produkt enthält 46,5 Gew.% $P_2O_5$ und 45,5 Gew.% $Al_2O_3$. Dieses $AlPO_4$-5 wird mit Boehmit im Gewichtsverhältnis 60:40 zu 2 mm-Strängen verformt, bei 110 °C getrocknet und bei 500 °C/16 h calciniert.

Katalysator F

Siliciumaluminiumphosphat-5 (SAPO-5) wird hergestellt aus einer Mischung aus 200 g 98 %ige Phosphorsäure, 136 g Boehmit, 60 g Kieselsol (30 %ig), 287 g Tripropylamin und 587 g $H_2O$, Diese Mischung wird bei 150 °C während 168 Stunden unter autogenem Druck umgesetzt. Nach Filtration wird das kristalline Produkt bei 120 °C getrocknet und bei 500 °C calciniert. SAPO-5 enthalt 49,8 Gew.% $P_2O_5$, 33,0 Gew.% $Al_2O_3$, 6,2 Gew.% $SiO_2$. SAPO-5 wird mit einem Verstrangungshilfsmittel zu 3 mm-Strängen verformt, bei 120 °C getrocknet und bei 500 °C calciniert.

Katalysator G

Kommerziell erhältliches Zirkonphosphat $Zr_3(PO_4)_4$ in Reinsubstanz verformt.

Katalysator H

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3 mm-Strängen verformt. Diese Stränge werden bei 100 °C getrocknet und bei 350 °C calciniert. Katalysator H enthält 8,77 Gew.% B und 28,3 Gew.% P.

Katalysator I

$SiO_2$ im Handel erhältlich unter D 11-10®.

Die mit diesen Katalysatoren erzielten Versuchsergebnisse und Versuchsbedingungen sind in den Tabellen 1 und 2 zusammengefaßt.

Beispiel 16

Es werden 100 ml p-tert.-Butylphenylglycidsäuremethylester/Stunde in Gegenwart von 200 l/h $N_2$ und bei 180 °C über 1 l Katalysator A, der in einem von außen elektrisch beheizten Rohrreaktor untergebracht ist, geleitet. Die gasförmigen Reaktionsprodukte werden kondensiert und nach üblichen Methoden aufgearbeitet und charakterisiert. Die Reinherstellung des 3-[p-tert.-Butyl]phenylbrenztraubensäuremethylesters gelingt durch übliche Destillation. Es werden 91,8 % Ausbeute isoliert.

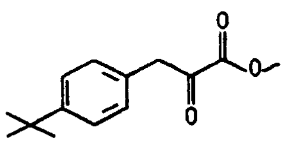

Kp = 135°C/0,3 mbar
weiße Kristalle

Beispiel 17

Es werden 100 ml des 3-[p-tert.-Butyl]phenylbrenztraubensäuremethylesters in Gegenwart von 200 l/h $N_2$ verdampft und bei 350 °C über 1 l Katalysator A (Apparatur wie bei Beispiel 16) geleitet. Nach üblicher Aufarbeitung findet man 31 %igen Umsatz und 83 % Selektivität für p-tert.-Butyl-phenylessigsäuremethylester.

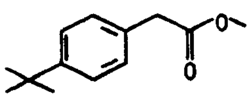

Kp = 96°C/0,4 mbar

Beispiel 18

Es wird analog Beispiel 16 gearbeitet jedoch bei 350 °C. Es wird eine Mischung aus 3-[p-tert.-Butyl]-phenylbrenztraubensäuremethylester und p-tert.-Butylphenylessigsäuremethylester im Verhältnis 65:35 gefunden.

Tabelle 1

| Umsetzung von Phenylglycidsäuremethylester (1) zu 3-Phenylbrenztraubenmethylester (2) | | | | | | | | | |
|---|---|---|---|---|---|---|---|---|---|
| Beispiele | 1 | 2 | 3 | 4 | 5 | 6 | 7 | 8 | 9 |
| Katalysatoren | A | B | C | D | E | F | G | H | I |
| Temperatur °C | 200 | 200 | 200 | 200 | 200 | 200 | 200 | 200 | 200 |
| WHSV $h^{-1}$ | 3,0 | 3,0 | 3,0 | 3,0 | 3,0 | 3.0 | 3,0 | 3,0 | 3,0 |
| Umsatz von (1) | 100 | 100 | 100 | 100 | 100 | 100 | 100 | 100 | 100 |
| Selektivität von (2) | 92,5 | 88,1 | 93,7 | 89,7 | 78,3 | 86,9 | 81,5 | 84,8 | 80,3 |

Tabelle 2

Umsetzung von substituierten Phenylglycidsäuremethylestern der Formel (II) zu substituierten 3-Phenylbrenztraubensäuremethylestern der Formel (I)

| Beispiel | 10 | 11 | 12 | 13 | 14 | 15 |
|---|---|---|---|---|---|---|
| Substituent an Aromaten | 4-Fluor- | 4-Methyl | 4-Methoxy- | 4-Trifluor-methyl | 4-t.-Butyl | 1-Methyl-4-Fluor |
| Katalysator | A | A | A | A | A | A |
| Temperatur °C | 200 | 200 | 200 | 200 | 200 | 200 |
| WHSV $h^{-1}$ | 3.0 | 3.0 | 3.0 | 3,0 | 3.0 | 3,0 |
| Umsatz von (II) | 100 | 100 | 100 | 100 | 100 | 100 |
| Selektivität von (I) | 87,5 | 93,7 | 92,5 | 92,3 | 90,3 | 91,0 |

## Patentansprüche

**1.** Verfahren zur Herstellung von α-Ketocarbonsäureestern

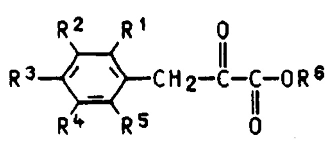

(I),

in der $R^1$ bis $R^5$ untereinander gleich oder verschieden sind und Wasserstoff oder geradkettige bzw. verzweigte Alkyl-, Alkenyl-, Hydroxyl-, Alkoxyl- und/ oder Cycloalkylreste und/oder Halogenreste bedeuten können und $R^6$ für einen unverzweigten Niederalkylrest steht, dadurch gekennzeichnet, daß man Glycidsäureester der Formel II

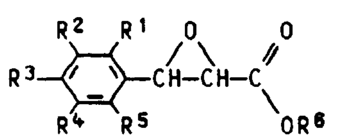

(II),

in der $R^1$ bis $R^6$ obige Bedeutung haben, in Gegenwart von Zeolithen und/oder Phosphaten und/oder Phosphorsäure bzw. Borsäure auf Trägermaterial und/oder sauren Metalloxiden als Katalysatoren umsetzt.

2. Verfahren nach Anspruch 1, dadurch gekennzeichnet, daß man als Katalysatoren Aluminium-, Boro- und Eisensilikatzeolithe des Pentasiltyps, Zeolithe des Faujasittyps oder deren Gemische verwendet.

3. Verfahren nach Anspruch 1 und 2, dadurch gekennzeichnet, daß man mit Alkalimetallen, Übergangsmetallen, Seltenen Erdmetallen oder deren Gemische dotierte Zeolithe als Katalysatoren verwendet.

4. Verfahren nach Anspruch 1, dadurch gekennzeichnet, daß man als Katalysatoren Phosphate der Elemente B, Al, Zr, Ce, Fe, Sr oder deren Gemische verwendet.

5. Verfahren nach Anspruch 1, dadurch gekennzeichnet, daß man als Katalysatoren hydrothermal hergestellte Phosphate mit Zeolithstruktur verwendet.

6. Verfahren nach Anspruch 1, dadurch gekennzeichnet, daß man als Katalysatoren hydrothermal hergestellte Aluminiumphosphate oder Siliciumaluminiumphosphate oder Siliciumeisenaluminiumphosphate oder Boraluminiumphosphate verwendet.

7. Verfahren nach Anspruch 1, dadurch gekennzeichnet, daß man als Katalysatoren Phosphorsäure bzw. Borsäure auf Trägermaterialien $SiO_2$, $Al_2O_3$, $TiO_2$ und Bims verwendet.

8. Verfahren nach Anspruch 1, dadurch gekennzeichnet, daß man als Katalysatoren Metalloxide der Elemente Al, Si, Ti, Zr, Be, Cr und V verwendet.

9. Verwendung der nach Anspruch 1 erhaltenen α-Ketocarbonsäureester zur Herstellung von Phenylessigsäureestern durch Decarbonylierung in Gegenwart von Zeolithen und/oder Phosphaten und/oder Phosphorsäure bzw. Borsäure auf Trägermaterial und/oder sauren Metalloxiden als Katalysatoren bei Temperaturen über 300 °C.

## Claims

1. A process for preparing α-ketocarboxylic esters

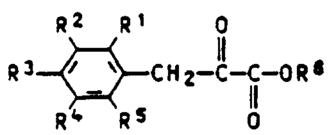

(I)

where $R^1$, $R^2$, $R^3$, $R^4$ and $R^5$ are identical to or different from one another and are each hydrogen or straight-chain or branched alkyl, alkenyl, hydroxyl, alkoxy, cycloalkyl or halogen and $R^6$ is unbranched lower alkyl, which comprises reacting glycidic esters or the formula II

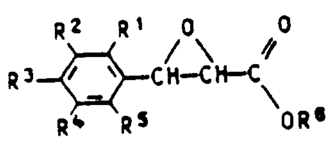

(II)

where $R^1$, $R^2$, $R^3$, $R^4$, $R^5$ and $R^6$ are each as defined above, in the presence of zeolites and/or phosphates and/or phosphoric acid or boric acid on a carrier material and/or acidic metal oxide as catalysts.

2. A process as claimed in claim 1, wherein the catalysts used are aluminosilicate, borosilicate or iron silicate zeolites of the pentasil type, zeolites of the faujasite type or mixtures thereof.

3. A process as claimed in claim 1 and 2, wherein zeolites doped with alkali metals, transition metals, rare earth metals or mixtures thereof are used as catalysts.

4. A process as claimed in claim 1, wherein the catalysts used are phosphates of the elements B, Al, Zr, Ce, Fe or Sr or mixtures thereof.

5. A process as claimed in claim 1, wherein the catalysts used are hydrothermally synthesized phosphates having a zeolite structure.

6. A process as claimed in claim 1, wherein the catalysts used are hydrothermally synthesized aluminum phosphates or silicon aluminum phosphates or silicon iron aluminum phosphates or boron aluminum phosphates.

7. A process as claimed in claim 1, wherein the catalysts used are phosphoric acid or boric acid on $SiO_2$, $Al_2O_3$, $TiO_2$ and pumice as carrier materials.

8. A process as claimed in claim 1, wherein the catalysts used are metal oxides of the elements Al, Si, Ti, Zr, Be, Cr and V.

9. Use of the $\alpha$-ketocarboxylic esters obtained as claimed in claim 1 for preparing phenylacetic esters by decarbonylation in the presence of zeolites and/or phosphates and/or phosphoric acid or boric acid on a carrier material and/or acidic metal oxides as catalysts at above 300 °C.

## Revendications

1. Procédé de fabrication d'esters d' acides $\alpha$-cétocarboxyliques de la formule suivante

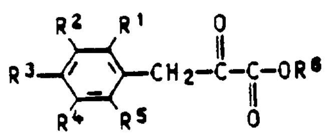

(I),

dans laquelle les symboles $R^1$ à $R^5$ sont mutuellement identiques ou différents et représentent, chacun, un atome d'hydrogène ou un radical cycloalkyle, alcoxy, hydroxyle, alcényle et/ou alkyle, à chaîne droite ou à chaîne ramifiée et/ou un radical halogène et $R^6$ represente un groupe alkyle inférieur non ramifié, caractérisé en ce que l'on fait réagir des esters d'acides glycidiques de la formule II

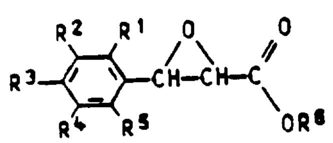

(II),

dans laquelle $R^1$ à $R^6$ possèdent les significations qui leur ont été attribuées ci-dessus, en présence de zéolites et/ou de phosphates et/ou d'acide phosphorique ou d'acide borique, sur un matériau de support et/ou des oxydes de métaux acides, servant de catalyseurs.

2. Procédé suivant la revendication 1, caractérisé en ce que l'on utilise, à titre de catalyseurs, des zéolites d'alumino-, boro- et sidéro-silicates du type pentasile, des zéolites du type faujasite, ou leurs mélanges.

3. Procédé suivant les revendications 1 et 2, caractérisé en ce que l'on utilise, à titre de catalyseurs, des zéolites dopées avec des métaux alcalins, des métaux de transition, des métaux des terres rares, ou leurs mélanges.

4. Procédé suivant la revendication 1, caractérisé en ce que l'on utilise, à titre de catalyseurs, des phosphates des éléments B, Al, Zr, Ce, Fe, Sr, ou leurs mélanges.

5. Procédé suivant la revendication 1, caractérisé en ce que l'on utilise, à titre de catalyseurs, des phosphates préparés par voie hydrothermique à structure zéolitique.

**6.** Procédé suivant la revendication 1, caractérisé en ce que l'on utilise, à titre de catalyseurs, des phosphates de bore et d'aluminium, des phosphates de silicium, de fer et d'aluminium, des phosphates de silicium et d'aluminium, ou des phosphates d'aluminium, préparés par voie hydrothermique.

**7.** Procédé suivant la revendication 1, caractérisé en ce que l'on utilise, à titre de catalyseurs, de l' acide phosphorique, ou de l'acide borique, sur des matériaux de support que sont $SiO_2$, $Al_2O_3$, $TiO_2$ et la pierre ponce.

**8.** Procédé suivant la revendication 1, caractérisé en ce que l'on utilise, à titre de catalyseurs, des oxydes de métaux des éléments Al, Si, Ti, Zr, Be, Cr et V.

**9.** Utilisation des esters d'acides $\alpha$-cétocarboxyliques obtenus suivant la revendication 1 en vue de la fabrication d'esters de l'acide phénylacétique par décarbonylation en présence de zéolites et/ou de phosphates et/ou d'acide phosphorique ou d'acide borique, sur un matériau de support et/ou des oxydes de métaux acides servant de catalyseurs, à des températures supérieures à 300 °C.